# EUROPEAN PATENT APPLICATION

(11) **EP 3 686 579 A1**
(43) Date of publication of application: **29.07.2020**
(21) Application number: 19382058.6
(22) Date of filing: 28.01.2019
(51) Int. Cl.: G01N 21/91, A61L 2/28, B01D 65/08, C12Q 1/22, G01N 21/94, B01D 61/02, A61B 90/70

(54) **METHOD FOR VISUALIZING AND QUANTIFYING BIOFILM ON SOLID SURFACES**

(71) Applicant: Dow Global Technologies Llc, Midland, MI 48674 (US)
(72) Inventor: MASSONS, Gerard, 43006 Tarragona (ES); GILABERT ORIOL, Guillem, 43006 Tarragona (ES); MOLINA, Veronica Garcia, Zurich (CH)
(74) Representative: Houghton, Mark Phillip

(57) **Abstract**

A method for visualizing biofilm on a solid surface. The method comprises steps of:
(a) providing a solid surface which has been in contact with an aqueous medium and at least a portion of the solid surface potentially is covered with biofilm,
(b) maintaining the solid surface in a horizontal position and covering the solid surface with an aqueous dispersion of carbon particles,
(c) tilting the solid surface at an angle of at least 5° from horizontal to allow excess aqueous dispersion of carbon particles to flow from the surface,
(d) detecting any biofilm present on the solid surface by determining whether there are areas not covered by aqueous dispersion of carbon particles.

## Description

### Background

This invention relates generally to a method for visualizing and quantifying biofilm on a solid surface, especially a polymeric surface.

Biofilm growth (biofouling) in Reverse Osmosis (RO) and Nanofiltration (NF) membrane elements still remains as one of the key challenges in RO/NF market. Blockage by bacteria-produced biofilm can cause an increase in pressure drop across the RO module, leading to hydraulic imbalance and possible damage to the module. Moreover, biofilms can affect membrane transport properties and create a drop in trans-membrane pressure (TMP) which lowers the flux. Each of these effects increases the energy of operation but also leads to frequent cleanings (CIP) to regain membrane performance. Typical methods involve staining the biofilm with a dye, which may be irreversible and also detrimental to the membrane. For example, N. Sreedhar et al., Desalination, 2018, 425:12-21, report a method using crystal violet to stain the membrane. This invention aims to provide a tool for enhanced visualization and quantification of biofilm structures, without permanent effect on either the biofilm structure or the membrane.

### Statement of Invention

The present invention is directed to a method for visualizing biofilm on a solid surface. The method comprises steps of:
(a) providing a solid surface which has been in contact with an aqueous medium and at least a portion of the solid surface potentially is covered with biofilm,
(b) maintaining the solid surface in a horizontal position and covering the solid surface with an aqueous dispersion of carbon particles,
(c) tilting the solid surface at an angle of at least 5° from horizontal to allow excess aqueous dispersion of carbon particles to flow from the surface,
(d) detecting any biofilm present on the solid surface by determining whether there are areas not covered by aqueous dispersion of carbon particles.

### Detailed Description

All percentages are weight percentages (wt%), and all temperatures are in °C, unless otherwise indicated. Averages are arithmetic averages unless indicated otherwise. All operations in the Examples were performed at room temperature (18 to 25 °C) unless specified otherwise. Preferably, the method of this invention is performed in the range from 3°C to 45°C, preferably 10°C to 35°C.

Preferably, the aqueous dispersion of carbon particles is an "India ink," a product sold under this name which is an aqueous dispersion of carbon particles. Preferably, the carbon particles have an arithmetic average diameter no greater than 2 micron; preferably no greater than 1 micron, preferably no greater than 0.7 micron. Preferably the particles have an arithmetic average diameter of at least 0.01 micron, preferably at least 0.05 micron; preferably at least 0.1 micron. The upper and lower limits are combinable. Preferably, the carbon content of the aqueous dispersion is from 0.5 to 10 wt%, preferably from 1 to 9 wt%, preferably from 2 to 8 wt%. The aqueous dispersion may contain small amounts of other substances, e.g., binders, surfactants, etc.

Preferably, the solid surface is a polymeric surface, preferably a membrane, e.g., a reverse osmosis, nanofiltration or hyperfiltration membrane. Preferably, the polymer forming the polymeric surface is a polyamide (e.g., comprising polymerized units of m-phenylene diamine or piperazine and trimesoyl chloride), polyester (e.g., polyethylene terephthalate) or polyolefin; preferably a polyamide.

Preferably, the aqueous dispersion is applied to a solid surface in a horizontal position, and the surface is then tilted to remove excess ink. Preferably, a small amount of ink is placed on a corner of the polymeric surface which is in a horizontal position. Preferably, the amount of ink added to the surface, based on area of the surface, is at least 0.1 mL/cm², preferably at least 0.11 mL/cm², preferably at least 0.12 mL/cm², preferably at least 0.13 mL/cm². The maximum amount of ink is not critical because larger amounts than needed will simply run off from the surface when it is tilted. Typically, no more than 0.2 mL/cm² is needed. Preferably, the surface is tilted so as to cause ink to flow in the direction liquid flowed over the surface when in use. Preferably, the surface is tilted at an angle of at least 20° from horizontal, preferably at least 30°; preferably no more than 80°, preferably no more than 60°, preferably no more than 50°, preferably no more than 40°. Preferably, the surface is tilted for a time from 2 to 40 seconds, preferably at least 5 seconds, preferably at least 10 seconds; preferably no more than 30 seconds, preferably no more than 20 seconds. Preferably, area of the surface which is with a biofilm is determined by calculation from visual observation, preferably with the aid of digital photography and digital image processing.

Preferably, image processing for surface biofilm quantification involves the following steps:
(a) Process the digital image, preferably with the aid of image processing software
(b) Convert the image to 8-bit grayscale, preferably by splitting color channels to RGB space, then preferably selecting the green layer and transforming it to 8-bit grayscale
(c) Transform the image to 1-bits, preferably by adjusting the black threshold from 255 to the color ranges when the first derivative of pixel count per color ranges is equal or lower than 0 (with a simple moving average of 10 periods).
(d) The biofilm surface coverage percentage is calculated by dividing the biofilm pixels (white pixels) to the total pixel count (PT) in the 1-bit space

Preferably, the membrane is illuminated from below to enhance the contrast and visualization of the areas where biofouling is present, which appear as clear and bright spots. Preferably, the polymeric surface to be tested has an average lightness of at least 100 lux preferably at least 300 lux; preferably no more than 4000 lux, preferably no more than 3000 lux.

### Examples

For a macro visual examination of samples dyed, a Leica MS5 stereo microscope was used, with magnification from 0.63x to 4x, allowing for realistic visualization of samples. The instrument uses two separate optical paths with two objectives and eyepieces. The result is slightly different viewing angles to produce a three-dimensional visualization of the sample. It is also capable of transmitted light illumination as well, by having a bulb beneath the object. Illumination is used as it enhances the contrast and visualization of the areas were biofouling is present. The area of the sample captured with the camera can vary from 7.6 to 510.7 mm², depending upon magnification level used in the stereo microscope. The resolution of the pictures obtained using a 12-megapixel digital camera for image acquisition is estimated to be around 10 µm, which is consistent with the diffraction-limit of the Leica MS5 stereo microscope. The technique is based on separating on a 1-bit image, the pixels corresponding to biofilm (white) from the pixels from the background (black).
(a) The biofouling samples used for validating this method were obtained from RO coupons suffering from biofouling. A wet sample of 4 cm x 4 cm from the RO coupons was placed horizontally in a Petri dish. A double side tape was used to hold together the membrane sample to the Petri dish. 2 mL of PELIKAN Black Fount India drawing ink (Pelikan, Switzerland) (arithmetic average particle size: 0.4 micron) was placed on the side of the membrane that had been in contact with the raw water that was filtered.
(b) After pouring the ink, the sample was decanted at 30° from horizontal for 10 seconds to remove excess ink
(c) The biofouled sample was visualized with the aid of a stereo microscope (Leica MS5). The stereo microscope magnification used was 2x with a light intensity of 500 lux
(d) A digital picture was obtained using a 12.1-megapixel digital camera (Canon Digital Ixus 200 IS) and processed using ImageJ™ 1.51 software
(e) The color was split to RGB channels. Then green layer was selected and was converted to 8-bit grayscale
(f) The image was transformed to 1-bits picture by adjust the black threshold to 110, this created an image where biofilm coverage could be visualized. The image contained 2,151,502 white pixels out of 12,000,000 pixels which represented an 18% biofilm surface coverage

## Claims

1. A method for visualizing biofilm on a solid surface; said method comprising steps of:
(a) providing a solid surface which has been in contact with an aqueous medium and at least a portion of the solid surface potentially is covered with biofilm,
(b) maintaining the solid surface in a horizontal position and covering the solid surface with an aqueous dispersion of carbon particles,
(c) tilting the solid surface at an angle of at least 5° from horizontal to allow excess aqueous dispersion of carbon particles to flow from the surface,
(d) detecting any biofilm present on the solid surface by determining whether there are areas not covered by aqueous dispersion of carbon particles.

2. The method of claim 1 in which the solid surface is a polymeric surface.

3. The method of claim 2 in which the aqueous dispersion of carbon particles is an India ink.

4. The method of claim 3 in which the biofilm is detected by obtaining a photographic image of biofilm comprising an aqueous dispersion of carbon particles.

5. The method of claim 4 in which the photographic image is processed digitally to produce a one-bit image.

6. The method of claim 5 in which the solid surface is tilted at an angle of at least 20° from horizontal.

7. The method of claim 6 in which the aqueous dispersion of carbon particles is added to the surface in an amount of at least 0.1 mL/cm².

8. The method of claim 7 in which the aqueous dispersion of carbon particles has a carbon content from 0.5 to 10 wt%.
